# EUROPEAN PATENT SPECIFICATION

(11) **EP 2 421 456 B1**
(45) Date of publication and mention of the grant of the patent: **09.09.2015**
(21) Application number: 10716442.8
(22) Date of filing: 23.04.2010
(51) Int. Cl.: A61B 17/82, A61B 17/84

(54) **System for minimally invasive crimp and cable for bone cerclage**
System zum minimalinvasiven Crimpen und Draht zum Umwickeln eines Knochens
Système pour cerclage osseux minimalement invasif utilisant un élément de sertissage et un câble

(30) Priority: 25.04.2009 US 172720 P
(43) Date of publication of application: 29.02.2012
(73) Proprietor: Synthes GmbH, 4436 Oberdorf (CH)
(72) Inventor: DELL'OCA, Alberto A. Fernandez, Montevideo 11500 (UY)
(74) Representative: Lusuardi, Werther
(86) International application number: PCT/US2010/032245
(87) International publication number: WO 2010/124211

(56) References cited:
- US-A- 5 569 253
- US-A- 5 653 711
- US-A- 6 017 347
- US-A1- 2008 208 223

## Description

### Field of the invention

The invention relates generally to surgical apparatus for treating bones and more particularly to an apparatus for bone fixation using cable for a bone cerclage technique.

### Background information

Bones are often treated using cerclage techniques in which a wire or other cable is wrapped around a portion of bone to facilitate fixation or repair thereof. Cerclage procedures generally require that a wire or cable looped around the bone be secured tightly with current devices and methods requiring, adjacent the target portion of bone, at least one incision large enough to permit the insertion of bone cerclage tools for inserting and securing the cable around the bone. Specifically, these techniques generally require an incision large enough to permit the insertion and manipulation of a cable crimping tool capable of applying the force necessary to crush or deform a crimp over the cable. Such systems and methods are of limited utility in minimally invasive procedures. Other known orthopedic cerclage systems incorporate a cable and a screw extending perpendicular to a free end of the cable and tightened thereover to secure the cable in a desired configuration. However, these systems are also difficult to employ in minimally invasive procedures.

U.S. Patent Nos. 5,653,711 A and 6,017,347 A disclose apparatuses according to the preamble of claim 1.

### Summary of the invention

The present invention is directed to an apparatus and system for securing a cerclage member about a bone. The apparatus according to the present invention comprises a crimp including a first lumen extending therethrough from a first lumen proximal opening to a first lumen distal opening and being configured to slidably receive a cerclage member therein. A proximal portion of the first lumen comprises a first recess configured to receive an enlarged proximal end of the cerclage member. The crimp further comprises a second lumen extending parallel to the first lumen from a second lumen proximal opening to a second lumen distal opening. A distal portion of the second lumen comprises a second recess configured to slidably receive a sleeve provided over a distal end of a cerclage member. A first wall of the crimp adjacent the second lumen distal opening is angled with respect to a second wall of the crimp adjacent the first lumen distal opening to permit pivoting of the sleeve thereagainst as the sleeve is drawn thereagainst through the tensioning of the cerclage member drawn therethrough.

### Brief description of the drawings

Fig. 1 shows a first perspective view of a crimp according to the present invention;
Fig. 2 shows a second perspective view of the crimp of Fig. 1;
Fig. 3 shows a third perspective view of the crimp of Fig. 1;
Fig. 4 shows a first partial cross-sectional view of the crimp of Fig. 1;
Fig. 5 shows a second partial cross-sectional view of the crimp of Fig. 1;
Fig. 6 shows a third partial cross-sectional view of the crimp of Fig. 1;
Fig. 7 shows a fourth partial cross-sectional view of the crimp of Fig. 1;
Fig. 8 shows a first perspective view of the crimp of Fig. 1 in a first operative configuration;
Fig. 9 shows a second perspective view of the crimp of Fig. 1 in a first operative configuration;
Fig. 10 shows a third perspective view of the crimp of Fig. 1 in a first operative configuration;

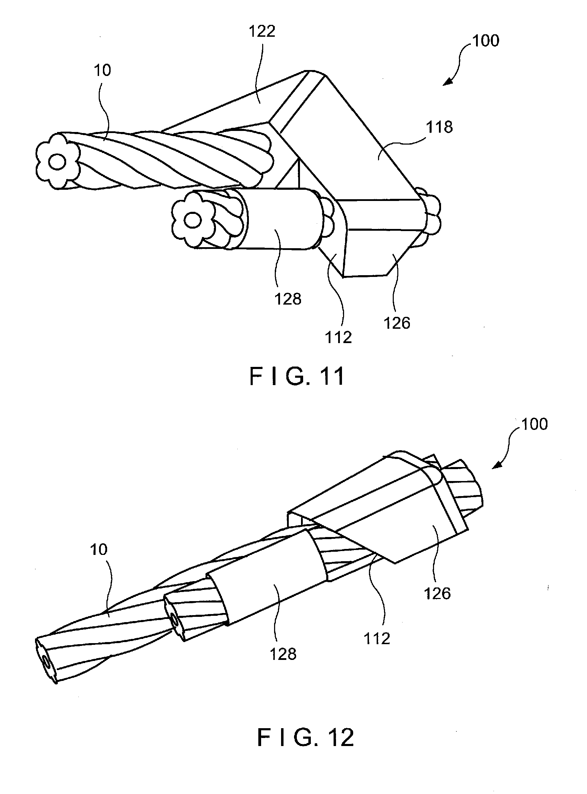

Fig. 11 shows a first perspective view of the crimp of Fig. 1 in a second operative configuration;
Fig. 12 shows a second perspective view of the crimp of Fig. 1 in a second operative configuration;

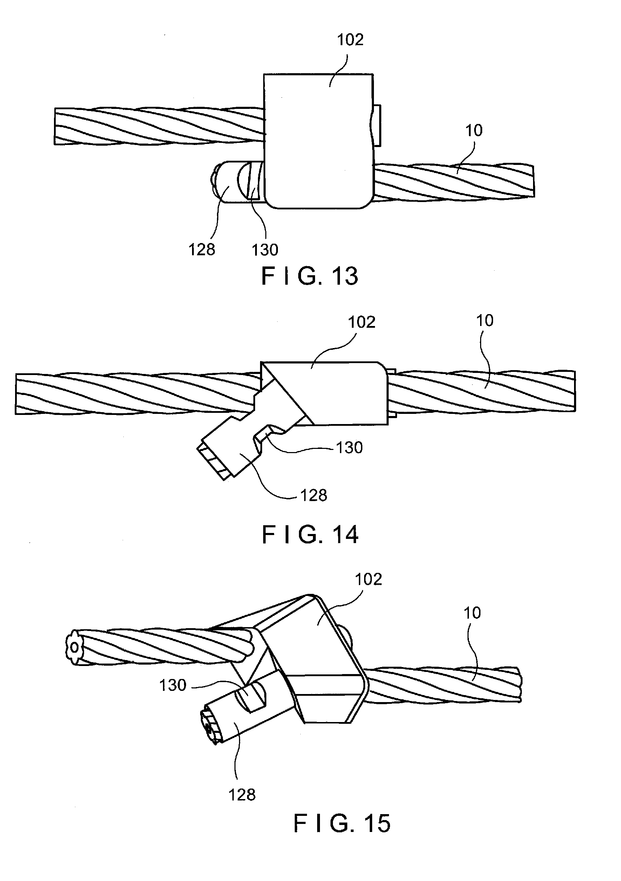

Fig. 13 shows a first perspective view of the crimp of Fig. 1 in a third operative configuration;
Fig. 14 shows a second perspective view of the crimp of Fig. 1 in a third operative configuration;
Fig. 15 shows a third perspective view of the crimp of Fig. 1 in a third operative configuration;
Fig. 16 shows a first view of a crimping tool; and
Fig. 17 shows a second view of the crimping tool of Fig. 16.

### Detailed Description

The present invention may be further understood with reference to the following description and the appended drawings, wherein like elements are referred to with the same reference numerals. The present invention relates generally to devices for the stabilization and fixation of fractured bones and bone fragments via a minimally invasive cerclage system. Specifically, the present invention relates to devices for securing a cerclage wire or cable in a desired position around a target portion of bone. Embodiments of the present invention may also be employed with any of a plurality of treatment procedures involving cerclage. As used in this application, the terms proximal and distal refer to a direction along the cerclage cable with a first end of the cable being identified as the proximal end and a second end of the cable being identified as distal. Components of an exemplary crimp according to the present invention will then be described in relation to the direction in which the cable passes therethrough. For example, an opening to a lumen into which a distal end of the cable is inserted and against which a proximal end of the cable is held will be described as the proximal opening of the lumen. The opposite end of the lumen from which the distal end of the cable exits will be described as the distal opening of the lumen and the other elements of the crimp will be similarly identified.

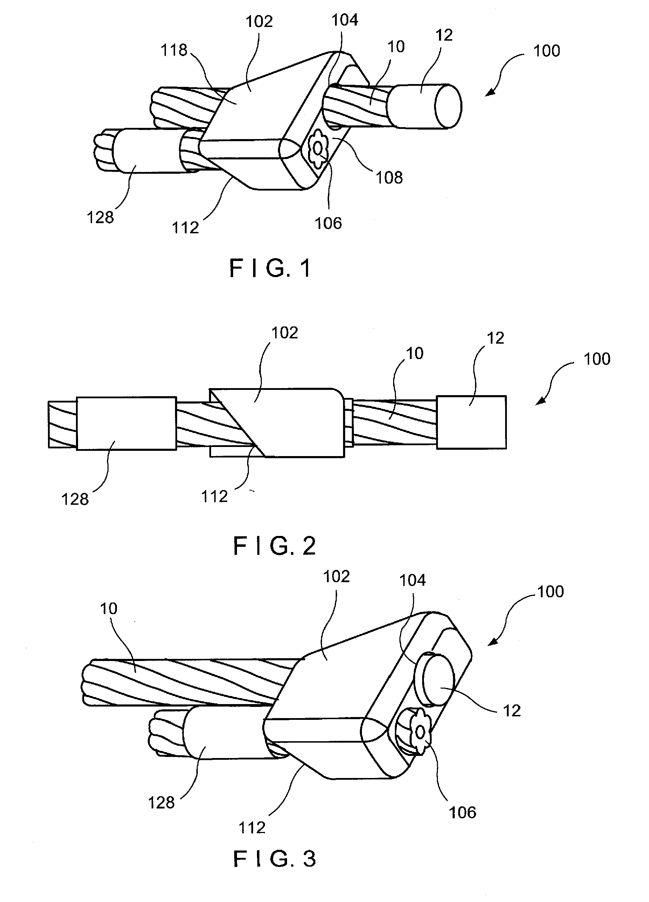

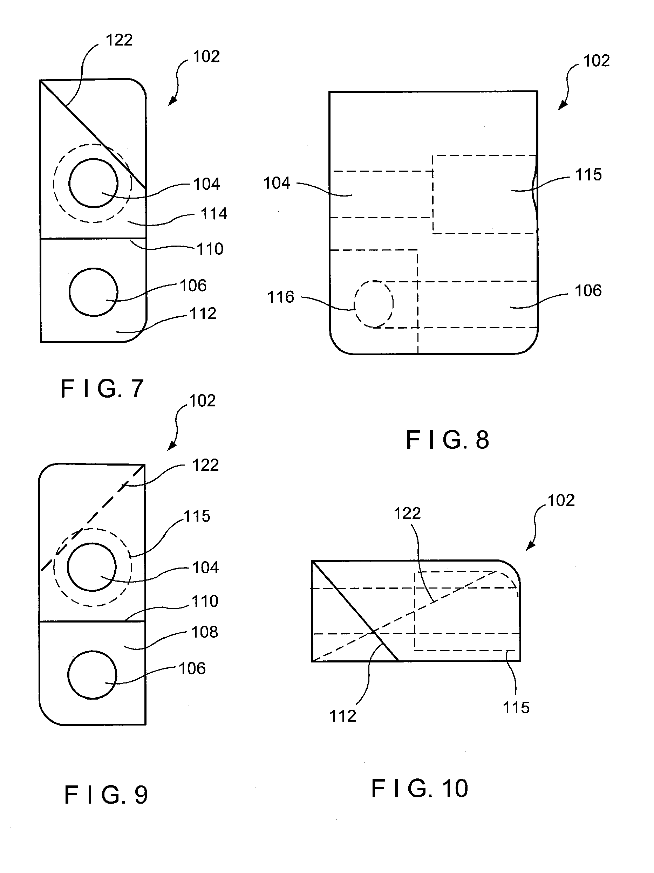

As shown in Figs. 1 - 3, a crimping device 100 according to a first exemplary embodiment of the invention comprises a crimp 102 for receiving a cable 10 to be looped around a target portion of bone (not shown). The crimp 102 defines first and second lumens 104, 106 extending therethrough from a first substantially planar face 108 to a second face 110 substantially parallel to one another. The second face 110 comprises a first beveled surface 112 adjacent an opening to the second lumen 106 angled relative to a longitudinal axis of the second lumen 106 so that, when the crimp 102 is in a desired position, the beveled surface faces a bone over which the crimp 102 is positioned. A second surface 114 of the second face 110 adjacent an opening to the first lumen 104 extends substantially perpendicular to the longitudinal axes of the first and second lumens 104, 106. However, as would be understood by those skilled in the art, the angle of the second surface 114 relative to the longitudinal axes of the lumens 104, 106 may be varied as desired without departing from the scope of the invention. The second surface 114 extends substantially perpendicular to third and fourth lateral faces 118, 120 which extend between the first and second faces 108, 110. The first beveled portion 112 extends relative to the second surface 114 at an angle selected to permit pivoting and deformation of the cable 10 thereagainst when the cable 10 is to be crimped in a desired configuration, as will be described in greater detail later on. The diameters of the first and second lumens 104, 106 are substantially equal to one another and are selected to permit slidable insertion of the cable 10 therethrough with minimal lateral movement of the cable 10 therewithin. Specifically, the diameter of the first and second lumens 104, 106 is substantially equal to an outer diameter of the cable 10 plus a small clearance to permit the cable 10 to slide smoothly therethrough. As shown in the partial cross-sectional views of Figs. 7 - 10, a proximal portion of the first lumen 104 includes a first increased diameter recess 115 configured to receive therein a cable stop 12 at a proximal end of the cable 10. The first recess 115 extends into the first lumen 104 by a length substantially equivalent to a length of the cable stop 12 along the cable 10 and is preferably sized and shaped to match a profile of the cable stop 12. In this case, the first recess 115 and the cable stop 12 are substantially cylindrical. It is noted, however, that the first recess 115 and cable stop 12 may be formed with any shape so long as the profile of the cable stop 12 is greater than a diameter of the distal portion of the first lumen 104 to prevent the cable stop 12 and the proximal end of the cable 10 from being drawn distally through the first lumen 104. In an operative configuration, a distal end (not shown) of the cable 10 is inserted into a proximal end of the first lumen 104 and drawn distally therethrough until the cable stop 12 is positioned within the first recess 115 so that engagement of the cable stop 12 with a distal end of the first recess 115 prevents the proximal end of the cable 10 from being drawn through the crimp 102.

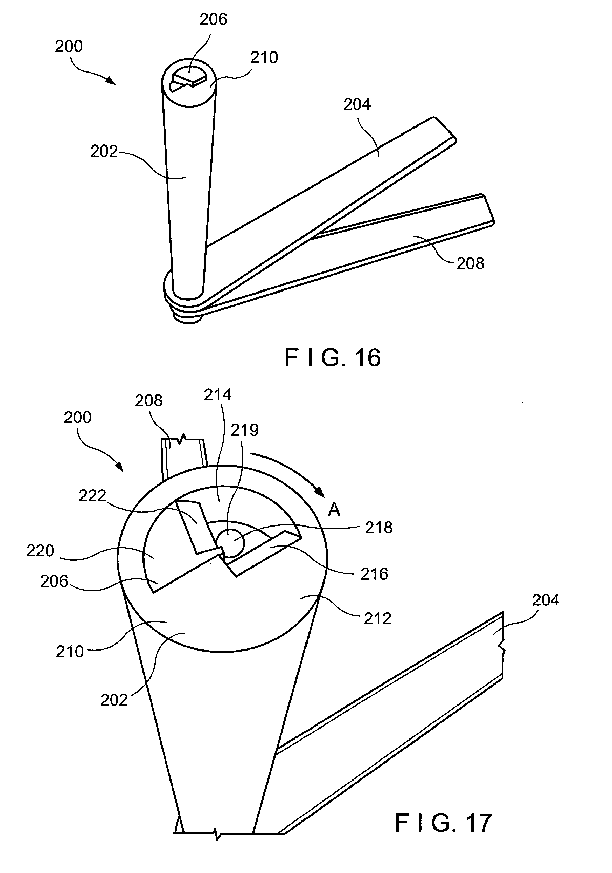

The beveled surface 112 is angled so that, as the sleeve 128 is drawn thereagainst through the tensioning of the cable 10 drawn therethrough, the sleeve 128 is pulled against the angled surface tilting it toward the bone over which the crimp 102 is positioned. The sleeve 128 is formed of a material which may be plastically deformed (e.g., by being crushed) to retain a deformed shape which will lock a portion of the cable extending therewithin relative to the sleeve 128. For example, the sleeve 128 may be formed of Stainless Steel, Titanium (Ti), Titanium Alloys, Cobalt Chromium (CoCr), plastics or bioresorbable materials (e.g., Magnesium). When the sleeve 128 is positioned as desired against the beveled surface 112 a desired tension is applied to the cable 10 as described below causing the sleeve 128 to tilt toward the bone over which the crimp 102 is placed. A crimping tool such as the tool 200 of Figs. 16-17 is then inserted through the minimally invasive incision and the sleeve 128 is crushed over the cable 10 to lock the cable 10 in a desired position and at a desired tension around a target portion of bone as will be described in more detail below.

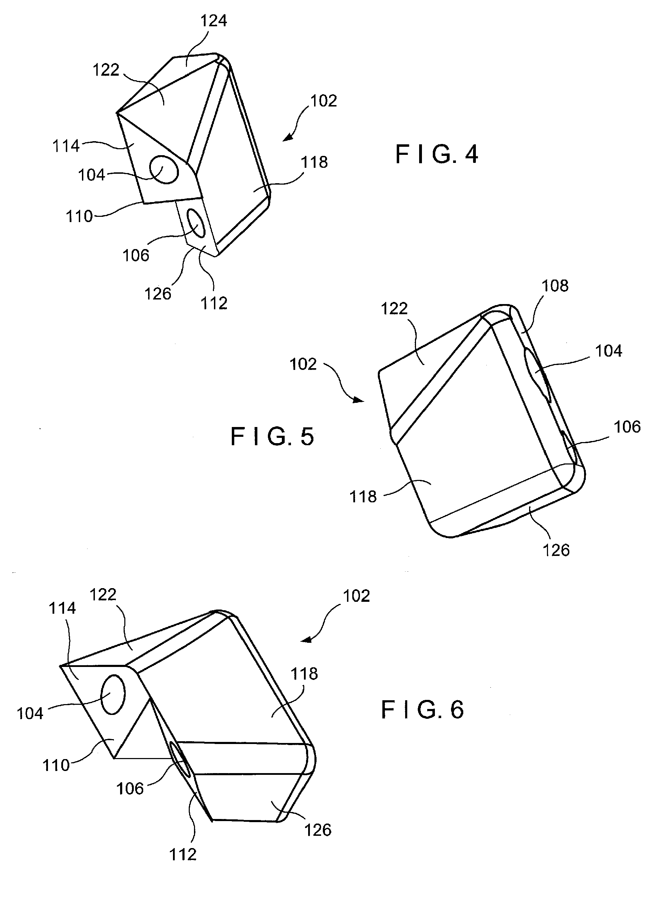

As shown in Fig. 8, the third and fourth lateral faces 118, 120 extending between the first and second faces 108, 110 are substantially parallel to one another. Fifth and sixth lateral faces 124, 126 extend between and are substantially perpendicular to the third and fourth faces 118, 120. The third face 118 further comprises a second beveled portion 122 extending over the fifth face 124 to connect to the fourth face 120, as shown in Figs. 4 - 9. As those skilled in the art will understand, the second beveled portion 122 reduced the outer profile of the crimp 102 while also reducing tissue irritation caused by the crimp 102.

The cable 10 according to an exemplary embodiment is initially positioned within the first lumen 104 with the cable stop 12 seated within the first recess 114. As those skilled in the art will understand, target portions of bone (not shown) are moved relative to one another into a desired corrective position (e.g., by reducing a fracture) prior to insertion of the cable 10 into the body. Once the target portions of bone have been positioned as desired, the crimp 102 and the cable 10 are inserted through a minimally invasive incision formed adjacent the target portions and the crimp 102 is positioned over one of the target portions. Specifically, the minimally invasive incision may be 2.54-5.08 cm. in length although smaller incisions are also envisioned without deviating from the scope of the present invention. The crimp 102 is approximately 10 mm wide and 10 mm. long, dimensions thereof being selected to ensure a minimal outer profile when seated over the bone. The cable 10 is then wound around the target portions of bone to hold the target portions of bone together as desired and the distal end of the cable 10 is inserted into a channel 130 in the sleeve 128 and slid proximally over the cable 10 into the second recess 116.

A crimping tool 200, as shown in Figs. 16 - 17, may then be positioned over the free end of the cable 10. Specifically, the crimping tool 200 comprises an outer substantially cylindrical member 202 having a first handle 204 at a proximal end thereof and an inner substantially cylindrical member 206 having a second handle 208 at a proximal end thereof. The inner cylindrical member 206 is configured and dimensioned to be positioned within the outer cylindrical member 202 and remain rotatable relative thereto via rotation of the first and second handles 204, 208. A distal end 210 of the outer cylindrical member 202 comprises a first end wall 212 extending substantially perpendicularly to a longitudinal axis of the outer cylindrical member 202 and extending into a channel 214 extending through the outer cylindrical member 202 by a predetermined distance. In one embodiment, the first end wall 212 may extend over approximately one half of a distal end of the channel 214 although this dimension may be changed as required to conform to the requirements of a procedure being performed and the dimensions of the cable 10. A first sharpened edge 216 is provided on the first end wall 212 to permit crimping and subsequent clipping of the sleeve 128 and cable 10, as will be described in greater detail hereinafter. The inner member 206 comprises a lumen 218 extending therethrough configured to receive the cable 10 while preventing the sleeve 128 from being inserted therethrough. A second end wall 220 formed on a distal end of the inner member 206 is located proximally of the first end wall 212 and formed substantially similarly to the first end wall 212. The second end wall 220 further comprises a second sharpened edge 222 with a leading edge thereof facing the first sharpened edge 216. The first and second handles 204, 208 are configured such that, when in alignment with one another (i.e., positioned over one another), the first and second sharpened edges 216, 222 are located adjacent one another with a distal opening 219 of the lumen 218 sealed by the first end wall 212. In an operative configuration, the first and second handles 204, 208 are rotated out of alignment from one another by a predetermined angle sufficient to expose the distal end 219 of the lumen 218. The free end of the cable 10 may then be inserted through the lumen 218 so that the sleeve 128 is positioned between the crimping tool 200 and the bone (not shown). The cable 10 is then tensioned to a desired level as would be understood by those skilled in the art so that a desired force is applied to the bone. As the desired force is being applied to the cable 10, the first and second handles 204, 208 are brought into alignment by, for example, rotating the second handle 208 in the direction A toward the position of the first handle 204. This movement causes the second sharpened edge 222 to move in the direction A, so that the sleeve 128 is compressed by the first and second end walls 212, 220. This compression causes a deformation of the sleeve 128 against the first beveled portion 112 and angles the sleeve 128 toward the target portions of bone. Specifically, this force simultaneously drives the sleeve 128 against the crimp 102 and against the bone so that an outer profile of the sleeve 128 relative to the bone is minimized. As described earlier, compression of the sleeve 128 against the bone (not shown) reduces an outer profile of the crimping device 100, thereby reducing potential soft tissue irritation by the sleeve 128. After the sleeve 128 has been deformed and the cable 10 is locked in place, the portion of the cable 10 extending distally from the sleeve 128 is cut to lie flush against the sleeve 128.

It will be apparent to those skilled in the art that various modifications and variations may be made in the structure and the methodology of the present invention, without departing from the scope of the invention. Thus, it is intended that the present invention cover modifications and variations of the invention provided that they come within the scope of the appended claims.

## Claims

1. An apparatus for securing a cerclage member (10) about a bone, comprising:
a crimp (102) including a first lumen (104) extending longitudinally therethrough from a first lumen proximal opening to a first lumen distal opening, the first lumen sized and shaped to slidably receive the cerclage member therein, a proximal portion of the first lumen comprising a first recess (115) sized and shaped to receive an enlarged proximal end of the cerclage member, the crimp further comprising a second lumen (106) extending substantially parallel to the first lumen from a second lumen proximal opening to a second lumen distal opening, a distal portion of the second lumen comprising a second recesss (116) sized and shaped to slidably receive a sleeve (128) provided over a distal end of a cerclage member,
**characterized in that** first wall (112) of the crimp adjacent the second lumen distal opening is angled with respect to a second wall (110) of the crimp adjacent the first lumen distal opening to permit pivoting of the sleeve thereagainst as the sleeve is drawn thereagainst through the tensioning of the cerclage member drawn therethrough.

2. The apparatus of claim 1, wherein the first recess has an increased diameter relative to the first lumen.

3. The apparatus of claim 1, wherein the second recess has an increased diameter relative to the second lumen.

4. The apparatus of claim 1, wherein the first lumen and second lumen extend parallel to one another.

5. The apparatus of claim 1, wherein the first and second lumens are substantially cylindrical.

6. A system for securing a cerclage member about a bone, comprising:
an apparatus according to claim 1;
a cerclage member including an increased diameter stop member (12) at a proximal end, the stop member being configured for insertion into the first recess but prevented from entering the first lumen; and
a sleeve (128) configured for slidable movement over the cerclage member, the sleeve configured to be deformable when at least partially positioned in the second recess.

7. The system of claim 6, wherein the sleeve comprises a mechanically weakened portion defining a desired deformation location thereon.

8. The system of claim 6, further comprising a second bevel (122) located on the crimp member configured to minimize an outer profile of the crimp member.

9. The system of claim 6, wherein the first recess has an increased diameter relative to the first lumen and the second recess has an increased diameter relative to the second lumen.

10. The system of claim 6, further comprising a crimping tool (200) configured to deform the sleeve over the cerclage member, the crimping tool comprising a first tubular member having a first lumen extending therethrough and a first handle at a proximal end thereof and a second tubular member rotatably housed within the first tubular member, the second tubular member comprising a second lumen extending therethrough and configured to receive the cerclage member, the second tubular member comprising a second handle located at a proximal end thereof, wherein rotation of the first handle into alignment with the second handle causes first and second crimping faces located on respective distal ends of the first and second tubular members to apply a compressive crimping force to the sleeve and cerclage member.

11. The system of claim 10, wherein the first and second crimping faces extend substantially perpendicularly to longitudinal axes of the first and second tubular members.

12. The system of claim 11, wherein the first and second crimping faces comprise sharpened edges.

## Patentansprüche

1. Vorrichtung zum Befestigen eines Cerclageelements (10) um einen Knochen, umfassend:
eine Crimpverbindung (102), die ein erstes Lumen (104) umfasst, das sich longitudinal von einer proximalen Öffnung des ersten Lumens durch diese zu einer distalen Öffnung des ersten Lumens erstreckt, wobei das erste Lumen derart bemessen und geformt ist, dass es das Cerclageelement gleitbar darin aufnimmt, wobei ein proximaler Abschnitt des ersten Lumens eine erste Ausnehmung (115) umfasst, die so bemessen und geformt ist, dass sie ein vergrößertes proximales Ende des Cerclageelements aufnimmt, wobei die Crimpverbindung ferner ein zweites Lumen (106) umfasst, das sich im Wesentlichen parallel zum ersten Lumen von einer proximalen Öffnung des zweiten Lumens zu einer distalen Öffnung des zweiten Lumens erstreckt, wobei ein distaler Abschnitt des zweiten Lumens eine zweite Ausnehmung (116) umfasst, die so bemessen und geformt ist, dass sie eine Hülse (128) gleitbar aufnimmt, die über einem distalen Ende eines Cerclageelements vorgesehen ist,
**dadurch gekennzeichnet, dass** eine erste Wand (112) der Crimpverbindung, die an die distale Öffnung des zweiten Lumens angrenzt, relativ zu einer zweiten Wand (110) der Crimpverbindung angewinkelt ist, die an die distale Öffnung des ersten Lumens angrenzt, um ein Drehen der Hülse gegen diese zu ermöglichen, wenn die Hülse durch das Spannen des durch diese gezogenen Cerclageelements gegen diese gezogen wird.

2. Vorrichtung nach Anspruch 1, **dadurch gekennzeichnet, dass** die erste Ausnehmung einen in Bezug auf das erste Lumen vergrößerten Durchmesser aufweist.

3. Vorrichtung nach Anspruch 1, **dadurch gekennzeichnet, dass** die zweite Ausnehmung einen in Bezug auf das zweite Lumen vergrößerten Durchmesser aufweist.

4. Vorrichtung nach Anspruch 1, **dadurch gekennzeichnet, dass** sich das erste Lumen und das zweite Lumen parallel zueinander erstrecken.

5. Vorrichtung nach Anspruch 1, **dadurch gekennzeichnet, dass** das erste und das zweite Lumen im Wesentlichen zylindrisch sind.

6. System zum Befestigen eines Cerclageelements um einen Knochen, das umfasst:
eine Vorrichtung nach Anspruch 1;
ein Cerclageelement, das ein Anschlagelement (12) mit vergrößertem Durchmesser an einem proximalen Ende umfasst, wobei das Anschlagelement so konfiguriert ist, dass es in die erste Ausnehmung einsetzbar ist, ohne dabei in das erste Lumen eintreten zu können; und
eine Hülse (128), die so konfiguriert ist, dass sie gleitbar über das Cerclageelement bewegbar ist, wobei die Hülse so konfiguriert ist, dass sie verformbar ist, wenn sie mindestens teilweise in der zweiten Ausnehmung positioniert ist.

7. System nach Anspruch 6, **dadurch gekennzeichnet, dass** die Hülse einen mechanisch geschwächten Abschnitt umfasst, der eine gewünschte Verformungsstelle auf dieser definiert.

8. System nach Anspruch 6, welches zusätzlich eine zweite Schrägung (122) umfasst, die auf dem Crimpelement angeordnet und so konfiguriert ist, dass sie ein Außenprofil des Crimpelements minimiert.

9. System nach Anspruch 6, **dadurch gekennzeichnet, dass** die erste Ausnehmung einen in Bezug auf das erste Lumen vergrößerten Durchmesser aufweist und die zweite Ausnehmung einen in Bezug auf das zweite Lumen vergrößerten Durchmesser aufweist.

10. System nach Anspruch 6, welches zusätzlich ein Crimpwerkzeug (200) umfasst, das so konfiguriert ist, dass es die Hülse über dem Cerclageelement verformt, wobei das Crimpwerkzeug ein erstes rohrförmiges Element mit einem ersten Lumen, das sich durch dieses erstreckt, und einem ersten Griff an einem proximalen Ende davon und ein zweites rohrförmiges Element umfasst, das drehbar im ersten rohrförmigen Element aufgenommen ist, wobei das zweite rohrförmige Element ein zweites Lumens umfasst, das sich durch dieses erstreckt und das so konfiguriert ist, dass es das Cerclageelement aufnimmt, wobei das zweite rohrförmige Element einen zweiten Griff umfasst, der an einem proximalen Ende davon angeordnet ist, wobei eine Drehung des ersten Griffs in eine Ausrichtung mit dem zweiten Griff bewirkt, dass erste und zweite Crimpflächen, die auf den jeweiligen distalen Enden des ersten und des zweiten rohrförmigen Elements angeordnet sind, eine Crimpdruckkraft auf die Hülse und das Cerclageelement ausüben.

11. System nach Anspruch 10, **dadurch gekennzeichnet, dass** sich die ersten und zweiten Crimpflächen im Wesentlichen orthogonal zu Längsachsen des ersten und des zweiten rohrförmigen Elements erstrecken.

12. System nach Anspruch 11, **dadurch gekennzeichnet, dass** die ersten und zweiten Crimpflächen geschärfte Ränder umfassen.

## Revendications

1. Appareil de fixation d'un organe de cerclage (10) autour d'un os, comprenant :
un sertissage (102) incluant une première lumière (104) s'étendant longitudinalement à travers celui-ci à partir d'une ouverture proximale de la première lumière jusqu'à une ouverture distale de la première lumière, la première lumière étant dimensionnée et formée pour recevoir de façon coulissante l'organe de cerclage à l'intérieur, une partie proximale de la première lumière comprenant un premier évidement (115) dimensionné et formé pour recevoir une extrémité proximale agrandie de l'organe de cerclage, le sertissage comprenant en outre une deuxième lumière (106) s'étendant sensiblement parallèlement à la première lumière à partir d'une ouverture proximale de la deuxième lumière jusqu'à une ouverture distale de la deuxième lumière, une partie distale de la deuxième lumière comprenant un deuxième évidement (116) dimensionné et formé pour recevoir de façon coulissante un manchon (128) agencé sur une extrémité distale d'un organe de cerclage,
**caractérisé en ce qu'**une première paroi (112) du sertissage adjacente à l'ouverture distale de la deuxième lumière est inclinée par rapport une deuxième paroi (110) de sertissage adjacente à l'ouverture distale de la première lumière pour permettre un pivotement du manchon contre celle-ci lorsque le manchon est tiré contre celle-ci via la mise en tension de l'organe de cerclage tiré à travers celle-ci.

2. Appareil selon la revendication 1, dans lequel le premier évidement a un diamètre accru par rapport à la première lumière.

3. Appareil selon la revendication 1, dans lequel le deuxième évidement a un diamètre accru par rapport à la deuxième lumière.

4. Appareil selon la revendication 1, dans lequel la première lumière et la deuxième lumière s'étendent parallèlement l'une à l'autre.

5. Appareil selon la revendication 1, dans lequel les première et deuxième lumières sont sensiblement cylindriques.

6. Système de fixation d'un organe de cerclage autour d'un os, comprenant :
un appareil selon la revendication 1 ;
un organe de cerclage incluant un organe de butée à diamètre accru (12) au niveau d'une extrémité proximale, l'organe de butée étant configuré pour une insertion dans le premier évidement mais pour l'empêcher d'entrer dans la première lumière ; et
un manchon (128) configuré pour se déplacer par coulissement sur l'organe de cerclage, le manchon étant configuré pour être déformable lorsqu'il est au moins partiellement positionné dans le deuxième évidement.

7. Système selon la revendication 6, dans lequel le manchon comprend une partie mécaniquement fragilisée définissant un emplacement de déformation souhaité sur celui-ci.

8. Système selon la revendication 6, comprenant en outre un deuxième biseau (122) situé sur l'organe de sertissage, configuré pour minimiser un profil externe de l'organe de sertissage.

9. Système selon la revendication 6, dans lequel le premier évidement a un diamètre accru par rapport à la première lumière et le deuxième évidement a un diamètre accru par rapport à la deuxième lumière.

10. Système selon la revendication 6, comprenant en outre un outil de sertissage (200) configuré pour déformer le manchon sur l'organe de cerclage, l'outil de sertissage comprenant un premier organe tubulaire ayant une première lumière s'étendant à travers celui-ci et une première poignée au niveau d'une extrémité proximale de celui-ci et un deuxième organe tubulaire logé avec faculté de rotation au sein du premier organe tubulaire, le deuxième organe tubulaire comprenant une deuxième lumière s'étendant à travers celui-ci et configuré pour recevoir l'organe de cerclage, le deuxième organe tubulaire comprenant une deuxième poignée située au niveau d'une extrémité proximale de celui-ci, dans lequel une rotation de la première poignée en alignement avec la deuxième poignée amène les première et deuxième faces de sertissage situées sur des extrémités distales respectives des premier et deuxième organes tubulaires à appliquer une force de sertissage de compression sur le manchon et l'organe de cerclage.

11. Système selon la revendication 10, dans lequel les première et deuxième faces de sertissage s'étendent sensiblement perpendiculairement à des axes longitudinaux des premier et deuxième organes tubulaires.

12. Système selon la revendication 11, dans lequel les première et deuxième faces de sertissage comprennent des bords affûtés.
